Europäisches Patentamt

(19) European Patent Office

Office européen des brevets

(11) Publication number: **0 082 184**
**B1**

(12) **EUROPEAN PATENT SPECIFICATION**

(45) Date of publication of patent specification: **18.01.89**

(21) Application number: **82902222.7**

(22) Date of filing: **28.05.82**

(86) International application number:
**PCT/US82/00742**

(87) International publication number:
**WO 83/00085 20.01.83 Gazette 83/02**

(51) Int. Cl.⁴: **A 61 K 31/40**, A 61 K 31/195

(54) **NUTRITIONAL COMPOSITION FOR MANAGEMENT OF HEPATIC FAILURE.**

(30) Priority: **29.06.81 US 278914**

(43) Date of publication of application:
**29.06.83 Bulletin 83/26**

(45) Publication of the grant of the patent:
**18.01.89 Bulletin 89/03**

(84) Designated Contracting States:
**BE DE FR GB SE**

(56) References cited:
**US-A-3 832 465**
**US-A-3 950 529**
**US-A-4 025 650**
**US-A-4 053 589**
**US-A-4 259 353**
**US-A-4 279 917**

**Am.J.Clin.Nutrition 26, 916-925 (1973)**

**The file contains technical information submitted after the application was filed and not included in this specification**

(73) Proprietor: **BAXTER INTERNATIONAL INC.**
**One Baxter Parkway**
**Deerfield, IL 60015 (US)**

(72) Inventor: **MADSEN, David C.**
**600 Ardmore Terrace**
**Libertyville, IL 60048 (US)**
Inventor: **TUCKER, Hugh N.**
**3535 N. Janssen**
**Chicago, IL 60657 (US)**

(74) Representative: **MacGregor, Gordon et al**
**ERIC POTTER & CLARKSON 14 Oxford Street**
**Nottingham, NG1 5BP (GB)**

# EP 0 082 184 B1

**Description**

This invention relates to compositions and methods for nutritional management of hepatic (liver) failure. In particular the invention is directed at novel amino acid compositions designed to meet the altered metabolic needs of patients suffering from hepatic failure and the attendant derangements of normal amino acid metabolism characterized by this clinical condition.

Hepatic failure has numerous potential causes. Included among these causes are traumatic injuries to the organ and metabolic causes of a chronic (e.g., alcoholism) or acute nature (e.g., hepatitis, sepsis).

Of the numerous functions performed by the liver, one in particular has importance as the subject of the invention described below. The liver is the site of detoxification of numerous substances, in particular those nitrogenous wastes associated with protein metabolism. Especially important is the toxic by-product, ammonia ($NH_3$). The liver normally will detoxify ammonia by forming the nitrogen-containing substance urea (ureagenesis), which is then excreted via the kidneys.

When the liver is in various degrees of failure its ability to detoxify ammonia can become compromised. As a result ammonia can accumulate in the blood (hyperammonemia). It is a widely accepted belief among clinicians and researchers that hyperammonemia is dangerous, since ammonia is believed to be extremely toxic to the brain. A potential result of hyperammonemia is the onset or deepening of coma. When such coma is associated with hepatic failure it is termed "hepatic coma" or "hepatic encephalopathy".

Although the exact or immediate cause(s) of hepatic coma are not known with certainty, it is widely believed that ammonia can be a contributory factor. This is reflected in the fact that some of the non-nutritional therapeutic modalities employed in the clinical management of hepatic failure include treatments which have as their goal the reduction or elimination of ammonia input into the patient. Specifically, administration of the antibiotic, neomycin, and the synthetic carbohydrate, lactulose, are directed at reducing the production of ammonia by intestinal bacteria. It is known that the intestinal bacteria are the source of a substantial amount of ammonia, which reaches the blood-stream by intestinal absorption. Neomycin and lactulose are administered to reduce or eliminate this source of ammonia.

Current therapies for hepatic failure, with or without the complication of coma, are not generally nutritional in nature.

However, nutritional compositions which are disclosed to be tailored for hepatic failure are known. For example, see Ghadimi, U.S. Patent 3,832,405, Fischer et al, U.S. Patent 3,950,529 and West German Offenlegungsschrift 26 36 828.

It is known that certain amino acids (the L-forms of threonine, serine, tryptophan, glutamine, histidine, and glycine, hereafter termed "ammonotelic" amino acids) are catabolized by the body with the release of ammonia. (J. Rudman: Am. J. Clin. Nutrition; *26*, p. 916—925 (1973)).

It is an object of this invention to supply an amino acid composition which will reduce the ammonia produced endogenously (by the body, rather than by bacterial flora) by reducing the proportion of ammonotelic amino acids present in the nutritional source.

It is another object to ameliorate the hyperammonemia which accompanies hepatic dysfunction, thereby reducing the likelihood of a lapse into hepatic coma or a deepening of the comatose state.

It is an additional object to provide adequate nutrition to the hepatic diseased patent without compromising the foregoing two objects.

These and other objects will become apparent from the specification as a whole.

## Summary of the invention

We have discovered that the proportion of ammonotelic amino acids to other essential and nonessential amino acids must be carefully balanced. We believe that this proportion is critical to the successful therapy of the hepatic failure patient. Sufficient essential ammonotelic amino acids must be present to provide the required nutrients, but an excess of ammonotelic amino acids will exacerbate the clinical syndromes associated with liver disease, in particular hyperammonemia. Thus it is important that the proportion of ammonotelic amino acids not be reduced to a level at which nutrition is compromised, but that the level be sufficiently low as to not unnecessarily contribute to hyperammonemia. This is most readily accomplished by severely reducing or by eliminating L-serine, L-glycine and L-glutamine from mixtures of essential and nonessential amino acids to be used for nutritional support of liver diseased patients. The essential or semi-essential ammonotelic amino acids histidine, threonine and tryptophan are reduced to the lowest proportion compatible with effective nutritional support. The proportion of ammonotelic amino acids meeting these requirements has been found to be from 8 to 16 mole percent of the total amino acid composition.

## Detailed description of the invention

The compositions of this invention will contain the essential amino acids L-leucine, L-isoleucine, L-valine, L-lysine, L-threonine, L-methionine, L-phenylalanine and L-tryptophan. They may also contain the nonessential amino acids L-tryosine, L-arginine, L-proline, L-alanine and glycine. L-histidine is considered semi-essential, and in fact may be essential for neonates. For the purposes of nutrition in liver disease and this application, histidine will be denominated an essential amino acid.

2

EP 0 082 184 B1

L-serine and L-glutamine are the ammonotelic amino acids which are most readily absent from the compositions of this invention, even though serine has heretofore been included as a required amino acid in certain prior art nutritional amino acid compositions for liver disease, e.g., those of Fischer et al, U.S. Patent 3,950,529. Glycine is generally reduced to a low proportion of the total amino acids, ordinarily less than 8 mole % and preferably from 2 to 6 mole %. Glycine may be entirely absent from the compositions of this invention, but this is not preferred.

The essential ammonotelic amino acids histidine, threonine, and tryptophan are present in a proportion of from 6 to 16 mole %, ordinarily from 6 to 10 mole % and preferably 8 mole %. They are not entirely eliminated from the composition as this would be incompatible with the balanced nutritional objectives of the compositions described herein.

A typical composition of this invention will contain amino acids in the following proportions:

| Amino acids | Mole percent from | | |
|---|---|---|---|
| L-leucine | 19.4 | to | 19.8 |
| L-isoleucine | 16.2 | to | 16.4 |
| L-valine | 14.5 | to | 14.8 |
| L-lysine | 10.2 | to | 10.3 |
| L-methionine | 1.1 | to | 1.2 |
| L-phenylalanine | 0.7 | to | 0.9 |
| L-threonine | 2.3 | to | 3.9 |
| L-tryptophan | 0.5 | to | 0.7 |
| L-histidine | 2.7 | to | 2.8 |
| L-arginine | 8.5 | to | 8.7 |
| L-proline | 8.1 | to | 8.3 |
| glycine | 5.6 | to | 5.8 |
| L-analine | 8.1 | to | 8.3 |

The relative proportions of the amino acids in the above schedule may vary by as much as 15% of the mole percent ranges given, although in the most satisfactory composition the ranges will vary by no more than 5%.

The ratio of essential amino acids to nonessential amino acids by weight should range from 5:1 to 1:1. The essential amino acids should compromise from 60 to 75% by weight of the total amino acids in the composition.

The aromatic amino acids L-phenylalanine, L-tyrosine and L-tryptophan are ordinarily present at less than 8 mole %, preferably less than 3 mole % and optimally from 1 to 3 mole %.

The branched chain amino acids L-leucine, L-isoleucine and L-valine are present in a total of from 40—55% of the composition by weight, and preferably 50% by weight.

Cysteine is not included in the compositions of this invention since it has been found, contrary to the teachings of Ghadami (U.S. Patent 3,832,465), that the sulfur requirements of hepatic-diseased patients may be met by supplying L-methionine.

Whether the amino acid composition is administered parenterally or enterally will depend upon the clinical condition of the patient. If the gastrointestinal tract is sound the preferred administration route is enteral. Severely comatose patients will generally be fed parenterally. In non-comatose patients the composition may be administered as a supplement to normal oral nutrition. The suitable mode of administration will be within the skill of the ordinary artisan.

The composition may be supplemented with other nutrients such as vitamins, minerals, and biologically available, assimilable carbohydrates and fats. Supplementation may occur concomitantly with administration by premixing the additional nutrients with the amino acids and then administering, or by simultaneously supplying the nutrients by a separate administration route.

Parenteral compositions will ordinarily contain monosaccharides such as dextrose at typical infusion concentrations, e.g., from 10 to 40 percent by weight. Other carbohydrates such as oligosaccharides will be satisfactory. The solutions will be sterile and may contain stabilizers such as malate or sodium bisulfite. The concentration of amino acids in solutions for parenteral administration may range from 1 to 10 grams/dl (0.1 to 1.0 g/cm$^3$), and preferably is from 6 to 7 grams/dl (0.6 to 0.7 g/cm$^3$). The concentration is not critical, although as recognized by those skilled in the art the osmolarity of the solution should be compatible with the administration route (central or peripheral venous), and excessive water should not be given as carrier for the nutrients if elimination is perceived by the clinician to be a problem.

The enteral compositions are formulated particularly to provide the total nutritional requirements of the patient. Thus the Recommended Daily Dietary Allowances for water and fat soluble vitamins and for minerals are provided in a typical daily dosage of amino acids (1 g protein/day/kg of patient body weight) and calories (20 kcal/day/kg).

The composition and methods of this invention will be more fully understood by reference to the subsequent examples.

3

## EP 0 082 184 B1

Example 1

This contemplated example illustrates the use of an embodiment of the invention in an enteral feeding mode.

A 50 year old, 60 kg male with an exacerbation of hepatic failure due to acute cirrhosis of the liver from chronic alcohol ingestion, and with significant weight loss due to under-nutrition, exhibited protein intolerance, characterized in part by elevated serum ammonia values ($>100$ µg/dl; $>10$ µg/cm$^3$). The patient had a history of episodes of coma.

The composition to be administered to this patient had the following approximate analysis:

| | g/96g packet | %(W/W) | Kcal/ packet | Calories as % total |
|---|---|---|---|---|
| L-amino acids | 10.0 | 10.4 | 40 | 10.6 |
| Carbohydrate‡ | 73.2 | 76.3 | 292.9 | 77.4 |
| Fat* | 5.0 | 5.3 | 45.4 | 12.0 |
| Total nitrogen: | Approximately 1.55 g/3.4 oz (96 g) packet | | | |
| Total calories: | Approximately 378/3.4 oz (96 g) packet (338 nonprotein calories) | | | |
| Osmolarity: | At the usual dilution of 1.1 kcal/ml, the approximate osmolarity is 550 mOsm/l (osmolarity is 690 mOsm/kg water). | | | |

*Sunflower oil, medium chain triglycerides (fractionated coconut oil).
‡Glucose oligosaccharides, sucrose.

The amino acid, vitamin and mineral constituents were as follows:

| Amino acids | Amount (g) per 3.4 oz (96 g) packet (378 kcal) |
|---|---|
| L-leucine | 2.00 |
| L-isoleucine | 1.67 |
| L-valine | 1.33 |
| L-lysine-HCl (1.22 g) L-lysine-acetate (0.21 g) | free base 1.17 |
| L-threonine | 0.37 |
| L-methionine | 0.13 |
| L-phenylalanine | 0.10 |
| L-tryptophan | 0.10 |
| L-arginine | 1.17 |
| L-proline | .73 |
| L-alanine | .57 |
| glycine | .33 |
| L-histidine | .33 |

Vitamins/minerals

| | |
|---|---|
| Vitamin A | 167 R.E.** |
| Vitamin D | 33 I.U. |
| Vitamin E | 1.7 T.E.** |
| Vitamin C | 15 mg |
| Folic acid | 67 µg |
| Vitamin $B_1$ | 233 µg |
| Vitamin $B_2$ | 267 µg |
| Niacin | 3 mg |
| Vitamin $B_6$ | 367 µg |
| Vitamin $B_{12}$ | 0.5 µg |
| Biotin | 25 µg |
| Pantothenic acid | 0.9 mg |
| Vitamin $K_1$ | 17.5 µg |
| Choline | 67 mg |
| Sodium | 153 mg |
| Potassium | 392 mg |
| Calcium | 133 mg |
| Magnesium | 65 mg |
| Phosphorus | 165 mg |
| Chloride | 237 mg |
| Zinc | 2.5 mg |
| Iodine | 25 µg |
| Manganese | 417 µg |
| Iron | 3.0 mg |
| Copper | 333 µg |

**R.E.=Retinol equivalents; T.E.=Tocopherol equivalents

The 96 g packet of the above composition is mixed with 270 ml of water to yield approximately 350 ml. The solution is administered via a nasogastric tube.

The composition was diluted to half strength and fed for two days, working up to a feeding rate of full strength of approximately 20—24 grams of protein/hour in a total fluid volume of approximately 2,000 milliliters/day. Feeding was continuous over each 24-hour period. The patient's serum ammonia values decreased to a greater degree than ordinarily would be expected when compared to a diet containing a greater amount of ammonotelic amino acids.

The patient resumed taking meals after one week of enteral feeding with the above composition, after which the composition was used occasionally for supplementary nutrition.

Example 2

This contemplated example illustrates the use of an embodiment of the invention in a parenteral feeding mode. A 50 year old, 55 kg male with an acute exacerbation of hepatic failure due to cirrhosis of the liver secondary to chronic alcohol ingestion, and with significant weight loss due to under-nutrition, exhibited protein intolerance characterized in part by elevated serum ammonia (>100 µg/dl; >10 µg/cm³). The patient had a history of encephalopathy and was unable to tolerate enteral feedings.

The parenteral amino acid composition to be administered contained the following:

| | mg/dl (mg/10 cm³) |
|---|---|
| L-leucine | 1000 |
| L-isoleucine | 833 |
| L-valine | 667 |
| L-lysine (704) free base | 583 |
| L-methionine | 67 |
| L-phenylalanine | 50 |
| L-threonine | 183 |
| L-tryptophan | 50 |
| L-histidine | 167 |
| L-arginine | 583 |
| L-proline | 367 |
| glycine | 167 |
| L-alanine | 283 |

In addition the solution contained 20 mEq acetate and 11 mEa of chloride per liter. The pH was about 6.0 (by adjustment with acetic acid). About 3 mEq/l of sodium bisulfite was, added as a stabilizer. Concomitant administration of electrolytes and glucose for injection to provide approximately 40 kcal/kg (1500 ml of 50% Dextrose) completed the nutritional regimen. The rate of administration was 125 ml/hour by central venous catheter. The duration of this support was 10 days.

## Claims

1. A composition for administration to a patient having liver disease, comprising nonessential and essential amino acids including L-histidine but excluding cysteine, and wherein from 6 to 16 mole % of the composition consists of L-histidine, L-threonine and L-tryptophan taken together and the total proportion of L-histidine, L-threonine, L-tryptophan, L-glutamine and glycine is 8 to 16 mole %.

2. The composition of Claim 1 wherein the ratio of essential amino acids to nonessential amino acids by weight ranges from 5:1 to 1:1.

3. The composition of Claim 1 wherein the nonessential amino acids comprise greater than 20% and preferably from 60 to 75% by weight of the total amino acids in the composition.

4. The composition of Claim 1 wherein the mole % of L-serine, L-histidine, L-threonine, L-tryptophan, L-glutamine and glycine taken together ranges from 10 to 15 and preferably from 11 to 13.

5. The composition of Claim 1 containing L-leucine, L-isoleucine and L-valine in a total of 50% of the composition by weight.

6. A composition for administration to a patient having liver disease, comprising a cysteine and serine-free mixture of essential and nonessential amino acids in the following proportions:

| Amino acids | Mole percent from |
|---|---|
| L-leucine | 19.4 to 19.8 |
| L-isoleucine | 16.2 to 16.4 |
| L-valine | 14.5 to 14.8 |
| L-lysine | 10.2 to 10.3 |
| L-methionine | 1.1 to 1.2 |
| L-phenylalanine | 0.7 to 0.9 |
| L-threonine | 2.3 to 3.9 |
| L-tryptophan | 0.5 to 0.7 |
| L-histidine | 2.7 to 2.8 |
| L-arginine | 8.5 to 8.7 |
| L-proline | 8.1 to 8.3 |
| glycine | 5.6 to 5.8 |
| L-alanine | 8.1 to 8.3 |

7. The composition of claim 6 wherein the mole percentages of L-leucine, of L-isoleucine, L-valine, L-lysine, L-methionine, L-phenylalanine, L-threonine, L-tryptophan, L-histidine, L-arginine, L-proline, glycine and L-alanine are, respectively, 19.4, 16.2, 14.5, 10.2, 1.1, 0.8, 3.9, 0.6, 2.7, 8.5, 8.1, 5.6, and 8.1, preferably at an amino acid concentration of 1 to 10 grams/dl in aqueous solution.

8. The composition of Claim 6 wherein the mole percentages of L-leucine, L-isoleucine, L-valine, L-lysine, L-methionine, L-phenylalanine, L-threonine, L-tryptophan, L-histidine, L-arginine, L-proline, glycine and L-analine are respectively 19.8, 16.4, 14.8, 10.3, 1.2, 0.8, 2.3, 0.6, 2.8, 8.7, 8.3, 5.8, and 8.3, preferably at a concentration of 3 to 10 grams of amino acid/dl in sterile aqueous solution.

9. The composition of Claims 6, 7 or 8 further comprising vitamins, minerals, and digestively assimilable carbohydrates and fats.

10. The composition of any one of Claims 6 to 9 which is substantially free of electrolytes.

11. The composition of any one of Claims 6 to 10 comprising malate.

12. The composition of any one of Claims 6 to 11 comprising a sterilizing amount of sodium bisulfite.

## Patentansprüche

1. Zusammensetzung zur Verabreichung an Patienten mit Leberleiden, enthaltend nicht - essentielle und essentielle Aminosäuren einschließlich L-Histidin, aber ausschließlich Cystein, wobei 6 bis 16 Mol-% der Zusammensetzung aus L-Histidin, L-Threonin und L-Tryptophan zusammengenommen bestehen, und der Gesamtanteil an L-Histidin, L-Threonin, L-Tryptophan, L-Glutamin und Glycin 8 bis 16 Mol-% beträgt.

2. Zusammensetzung nach Anspruch 1, wobei das Gewichtsverhältnis von essentiellen Aminosäuren zu nichtessentiellen Aminosäure 5:1 bis 1:1 beträgt.

3. Zusammensetzung nach Anspruch 1, wobei die nichtessentiellen Aminosäuren mehr als 20 Gew.-% und vorzugsweise 60 bis 75 Gew.-% der gesamten Aminosäuren in der Zusammensetzung ausmachen.

4. Zusammensetzung nach Anspruch 1, wobei L-Serin, L-Histidin, L-Threonin, L-Tryptophan, L-Glutamin und Glycin zusammengenommen im Bereich von 10 bis 15 Mol-% und vorzugsweiset 11 bis 13 Mol-% liegen.

5. Zusammensetzung nach Anspruch 1, enthaltend L-Leucin, L-Isoleucin und L-Valin in einer Gesamtmenge von 50 Gew.-% der Zusammensetzung.

6. Zusammensetzung zur Verabreichung an Patienten mit Leberleiden, enthaltend ein Cystein- und Serin-freies Gemisch aus essentiellen und nicht-essentiellen Aminosäuren in folgenden Anteilen:

| Aminosäuren | Mol-% von |
|---|---|
| L-Leucin | 19,4—19,8 |
| L-Isoleucin | 16,2—16,4 |
| L-Valin | 14,5—14,8 |
| L-Lysin | 10,2—10,3 |
| L-Methionin | 1,1— 1,2 |
| L-Phenylalanin | 0,7— 0,9 |
| L-Threonin | 2,3— 3,9 |
| L-Tryptophan | 0,5— 0,7 |
| L-Histidin | 2,7— 2,8 |
| L-Arginin | 8,5— 8,7 |
| L-Prolin | 8,1— 8,3 |
| Glycin | 5,6— 5,8 |
| L-Alanin | 8,1— 8,3 |

7. Zusammensetzung nach Anspruch 6, wobei die Molprozentsätze von L-Leucin, von L-Isoleucin, L-Valin, L-Lysin, L-Methionin, L-Phenylalanin, L-Threonin, L-Tryptophan, L-Histidin, L-Arginin, L-Prolin, Glycin und L-Alanin 19,4; 16,2; 14,5; 10,2; 1,1; 0,8; 3,9; 0,6; 2,7; 8,5; 8,1; 5,6 bzw. 8,1 betragen, vorzugsweise bei einer Aminosäurekonzentration von 1 bis 10 g/dl in wässriger Lösung.

8. Zusammensetzung nach Anspruch 6, wobei die Molprozente von L-Leucin, L-Isoleucin, L-Valin, L-Lysin, L-Methionin, L-Phenylalanin, L-Threonin, L-Tryptophan, L-Histidin, L-Arginin, L-Prolin, Glycin und L-Alanin 19,8; 16,4; 14,8; 10,3; 1,2; 0,8; 2,3; 0,6; 2,8; 8,7; 8,3; 5,8 bzw. 8,3 betragen, vorzugsweise bei einer Konzentration von 2 bis 10 g Aminosäure/dl in steriler wässriger Lösung.

9. Zusammensetzung nach Anspruch 6, 7 oder 8, weiterhin enthaltend Vitamine, Mineralstoffe und verdauungsmäßig assimilierbare Kohlenhydrate und Fette.

10. Zusammensetzung nach einem der Ansprüche 6 bis 9, die im wesentlichen frei von Elektrolyten ist.

11. Zusammensetzung nach einem der Ansprüche 6 bis 10, enthaltend Malat.

12. Zusammensetzung nach einem der Ansprüche 6 bis 11, enthaltend eine sterilisierende Menge an Natriumbisulfit.

**Revendications**

1. Composition pour l'administration à un patient souffrant d'une maladie de foie, comprenant des aminoacides non essentiels et essentiels incluant la L-histidine mais excluant la cystéine, et dans laquelle de 6 à 16 moles % de la composition consistent en L-histidine, L-thréonine et L-tryptophane, considérés ensemble, et la proportion totale de L-histidine, L-thréonine, L-tryptophane, L-glutamine et glycine est de 8 à 16 mole %.

2. Composition suivant la revendication 1, dans laquelle le rapport pondéral des aminoacides essentiels aux aminoacides non essentiels est de 5:1 à 1:1.

3. Composition suivant la revendication 1, dans laquelle les aminoacides non essentiels représentent dans la composition plus de 20% et de préférence de 60 à 75% en poids des aminoacides totaux.

4. Composition suivant la revendication 1, dans laquelle le pourcentage molaire de L-sérine, L-histidine, L-thréonine, L-tryptophane, L-glutamine et glycine, considérés ensemble va de 10 à 15 et de préférence de 11 à 13.

5. Composition suivant la revendication 1, contenant les L-leucine, L-isoleucine et L-valine qui représentent au total 50% de la composition en poids.

6. Composition pour l'administration à un patient souffrant d'une maladie de foie, comprenant un mélange, qui est dépourvu de cystéine et de sérine, d'aminoacides essentiels et non essentiels, dans les proportions suivantes:

| Aminoacides | Pourcentage molaire |
| --- | --- |
| L-leucine | 19,4 à 19,8 |
| L-isoleucine | 16,2 à 16,4 |
| L-valine | 14,5 à 14,8 |
| L-lysine | 10,2 à 10,3 |
| L-méthionine | 1,1 à 1,2 |
| L-phénylalanine | 0,7 à 0,9 |
| L-thréonine | 2,3 à 3,9 |
| L-tryptophane | 0,5 à 0,7 |
| L-histidine | 2,7 à 2,8 |
| L-arginine | 8,5 à 8,7 |
| L-proline | 8,1 à 8,3 |
| glycine | 5,6 à 5,8 |
| L-analine | 8,1 à 8,3 |

7. Composition suivant la revendication 6, dans laquelle les pourcentages molaires de L-leucine, L-isoleucine, L-valine, L-lysine, L-méthionine, L-phénylalanine, L-thréonine, L-tryptophane, L-histidine, L-arginine, L-proline, glycine et L-alanine sont, respectivement, de 19,4; 16,2; 14,5; 10,2; 1,1; 0,8; 3,9; 0,6; 2,7; 8,5; 8,1; 5,6 et 8,1, de préférence à une concentration d'aminoacides de 1 à 10 g/dl en solution aqueuse.

8. Composition suivant la revendication 6, dans laquelle les pourcentages molaires de L-leucine, L-isoleucine, L-valine, L-lysine, L-méthionine, L-phénylalanine, L-thréonine, L-tryptophane, L-histidine, L-arginine, L-proline, glycine et L-analine sont, respectivement, de 19,8; 16,4; 14,8; 10,3; 1,2; 0,8; 2,3; 0,6; 2,8; 8,7; 8,3; 5,8 et 8,3, de préférence à une concentration de 3 à 10 grammes d'aminoacides par décilitre en solution aqueuse stérile.

9. Composition suivant les revendications 6, 7 ou 8, comprenant en outre des vitamines, des substances minérales, et, des carbohydrates et des graisses assimilables par digestion.

10. Composition suivant l'une quelconque des revendications 6 à 9, qui est sensiblement exempte d'électrolytes.

11. Composition suivant l'une quelconque des revendications 6 à 10, comprenant un malate.

12. Composition suivant l'une quelconque des revendications 6 à 11, comprenant une quantité stérilisante de bisulfite de sodium.